# EUROPEAN PATENT APPLICATION

(11) **EP 3 954 423 A1**
(43) Date of publication of application: **16.02.2022**
(21) Application number: 19924560.6
(22) Date of filing: 09.04.2019
(51) Int. Cl.: A61M 25/00

(54) **CATHETER**

(71) Applicant: ASAHI INTECC CO., LTD., Seto-shi, Aichi 489-0071 (JP)
(72) Inventor: TAKEMURA, Kazu, Seto-shi, Aichi 489-0071 (JP)
(74) Representative: Winter, Brandl - Partnerschaft mbB
(86) International application number: PCT/JP2019/015444
(87) International publication number: WO 2020/208702

(57) **Abstract**

To provide a catheter including a hollow shaft in which connected resin tubes are difficult to separate from one another at the joint parts.

A catheter 1 includes a hollow shaft 11 having one or more curved parts, in which at least one curved part W12 of the curved parts W1 has a first region R111 that is made of a first resin material AIM, a second region R112 that is provided closer to the distal end side in the longitudinal direction than the first region R111 and is made of a second resin material C1M having a different composition from the first resin material AIM, and a third region R113 that is provided to be adjacent to the first region R111 and the second region R112 and is made of a third resin material B1M formed by mixing the first resin material AIM and the second resin material C1M.

## Description

### TECHNICAL FIELD

The present invention relates to a catheter.

### BACKGROUND ART

As an instrument for guiding medical equipment such as a guide wire from the outside of a body into a body cavity such as a blood vessel, there is used a guiding catheter or the like, for example.

Such a catheter is normally formed so that the rigidity is gradually smaller toward the distal end side of the catheter so as to secure the body cavity followability and the kink resistance when the catheter is inserted into a blood vessel or the like curved in a complicated manner. As such a catheter whose rigidity is changed gradually, there is proposed, for example, a catheter in which a plurality of resin tubes having different rigidity are connected in series along the longitudinal direction (see Patent Literature 1, for example).

In the above-described catheter, the rigidity is smaller stepwisely, for example, toward the distal end side, which increases the body cavity followability and kink resistance, and the like.

### CITATION LIST

### Patent Literature

[Patent Literature 1] JP 2005-312633

### SUMMURY OF INVENTION

### Technical Problem

However, in the above-described conventional catheter, the body cavity followability and the kink resistance can be improved, while the connected resin tubes may be separated from one another at the joint parts.

In view of the above-described problem, the present invention aims at providing a catheter including a hollow shaft in which connected resin tubes are difficult to separate from one another at the joint parts.

### Solution to Problem

Some aspects of the present disclosure are: (1) a catheter, including a hollow shaft including one or more curved parts, in which at least one curved part of the curved parts has a first region that is made of a first resin material, a second region that is provided closer to a distal end side in a longitudinal direction than the first region and is made of a second resin material having a different composition from the first resin material, and a third region that is provided to be adjacent to the first region and the second region and is made of a third resin material formed by mixing the first resin material and the second resin material, (2) the catheter according to the above-described aspect (1), in which in the third region, a mixing rate of the second resin material relative to the first resin material is gradually larger from the first region toward the second region, and (3) the catheter according to the above-described aspect (1) or (2), in which a rigidity of a distal end portion of the hollow shaft is smaller than a rigidity of a proximal end portion of the hollow shaft positioned closer to a proximal end side than the distal end portion in the longitudinal direction.

Note that in the specification, the "distal end side" indicates a direction where the second region is positioned relative to the first region, along the longitudinal direction of a hollow shaft. Moreover, the "proximal end side" indicates a direction opposite from the distal end side, along the longitudinal direction of a hollow shaft. Moreover, the "distal end" indicates an end on the distal end side of an arbitrary member or region, and the "proximal end" indicates an end on the proximal end side of an arbitrary member or region.

### Advantageous Effects of Invention

The present invention is able to provide a catheter including a hollow shaft in which connected resin tubes are difficult to separate from one another at the joint parts.

### BRIEF DISCRIPTION OF DRAWINGS

FIG. 1 is a schematic side view illustrating a first embodiment of the invention.
FIG. 2 is a schematic side view illustrating a part of FIG. 1 in an enlarged manner.
FIG. 3 is a schematic side view illustrating a second embodiment of the invention.
FIG. 4 is a schematic side view illustrating a third embodiment of the invention.

### DESCRIPTION OF EMBODIMENTS

The catheter includes a hollow shaft including one or more curved parts, in which at least one curved part of the curved parts has a first region that is made of a first resin material, a second region that is provided closer to a distal end side in a longitudinal direction than the first region and is made of a second resin material having a different composition from the first resin material, and a third region that is provided to be adjacent to the first region and the second region and is made of a third resin material formed by mixing the first resin material and the second resin material.

Hereinafter, the first to third embodiments of the invention are described with reference to the enclosed drawings. However, the invention is not limited to the embodiments illustrated in the drawings. Moreover, the size of the catheter in each drawing is a size illustrated to facilitate understanding of the embodiments, and does not correspond to the actual size.

### [First Embodiment]

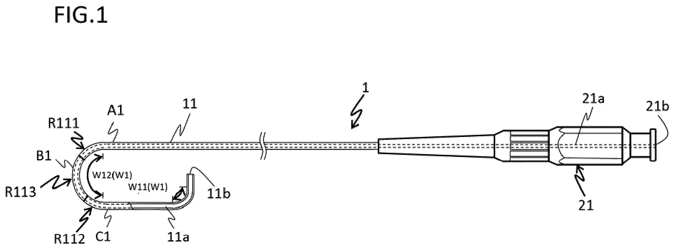

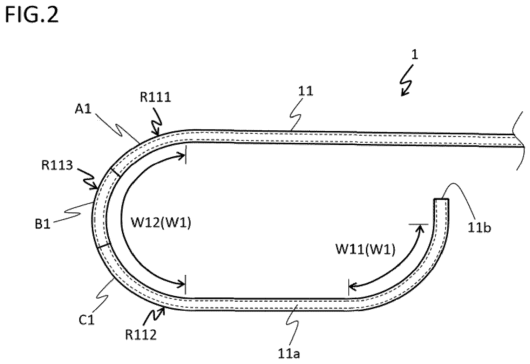

FIG. 1 and FIG. 2 are schematic side views illustrating the first embodiment of the invention. The catheter 1 schematically includes a hollow shaft 11 and a connector 21, as illustrated in FIG. 1 and FIG. 2.

The hollow shaft 11 is a hollow-shaped shaft having one or more curved parts. The hollow shaft 11 concretely includes a lumen 11a penetrating from an opening 11b at the distal end to the proximal end. A guide wire (not illustrated) or the like, for example, is inserted to the lumen 11a.

At least one curved part of the curved parts has a first region, a second region, and a third region. In the catheter 1, the hollow shaft 11 includes two curved parts W1 formed by a curved part W11 positioned on the distal end side (hereinafter, also referred to as a "distal end side curved part") and a curved part W12 positioned closer to the proximal end side than the distal end side curved part W11 (hereinafter, also referred to as a "proximal end side curved part"), and only the proximal end side curved part W12 of these two curved parts W11, W12 has the first to third regions R111 to R113.

To be more specific, the first to third regions at the proximal end side curved part W12 are the first region R111 configured by a part A1, the third region R113 configured by a part B1, and the second region R112 configured by a part C1, in this order from the proximal end side.

The first region is made of the first resin material. The second region is provided closer to the distal end side in the longitudinal direction than the first region, and is made of the second resin material having a different composition from the first resin material. In the catheter 1, concretely, the first region R111 (part A1) can be made of the first resin material (resin material AIM), and the second region R112 (part C1) can be made of the second resin material (resin material C1M), for example.

The resin materials AIM and C1M forming the part A1 and the part C1 preferably have antithrombogenicity, biocompatibility, and flexibility because the hollow shaft 11 is to be inserted in a body cavity such as a blood vessel, and there can be adopted a thermoplastic resin such as polyamide, polyolefin, polyester, polyurethane, silicone, and a fluororesin, for example. Note that from the viewpoint of improvement in operability of the catheter 1, the resin material C1M of the part C1 is normally a material having smaller rigidity than the resin material AIM of the part A1. Thus, the resin materials AIM and C1M can be appropriately combined from the above-described resin materials, for example.

The third region is provided to be adjacent to the first region and the second region, and is made of the third resin material formed by mixing the first resin material and the second resin material. In the catheter 1, the third region R113 (part B1) can be made of the third resin material (resin material B1M), for example.

As the third region R113, there can be adopted, for example, a region including only a single section of the same (homogeneous) composition, a region including a plurality of sections of different compositions connected in series, a region including a section of a composition changed gradually (monotonously), a region including the combination of these sections, and the like. In the embodiment, the third region R113 (section B1) is formed by only a single section of the same (homogeneous) composition.

As the third resin material B1M, there can be adopted the material formed by kneading the materials selected as the first and second resin materials AIM, C1M at a given ratio. Here, there can be adopted, as the given ratio, a single value in a case where the third region R113 includes only a single section of the same composition, a value for each section in a case where the third region R113 includes a plurality of sections of different compositions connected in series, a value changed gradually in a case where the third region R113 includes a section of a composition changed gradually (monotonously), or the like, for example.

Here, in a case where the third region R113 includes only a single section of the same composition, the first resin material A1M/second resin material C1M (mass ratio) is preferable to be 10/90 to 90/10, more preferable to be 30/70 to 70/30, even more preferable to be 40/60 to 60/40, and particularly preferable to be 50/50.

In a case where the third region R113 includes a plurality of sections of different compositions connected in series, it is preferable that the mixing ratio of the second resin material C1M relative to the first resin material AIM in the third region R113 is stepwisely larger for each section toward the distal end side.

In a case where the third region R113 includes a section of a composition changed gradually (monotonously), it is preferable that the mixing ratio of the second resin material C1M relative to the first region material AIM in the third region R113 is gradually larger from the first region R111 toward the second region R112 (toward the distal end side).

In this manner, with the above-described configuration of the mixing ratio of the first resin material AIM and the second resin material C1M in the third region R113, it is possible to reduce, as a whole, a composition difference of resins at the joint part between the first region R111 and the third region R113 and the joint part between the second region R112 and the third region R113, which enables firmer joint of resins having different materials.

The hollow shaft 11 can be obtained by joining an end of a tube (tubular member) made of the first resin material (resin material AIM), ends of a tube made of the third resin material (resin material B1M), and an end of a tube made of the second resin material (resin material C1M) in this order, for example. Moreover, in a case where the third region R113 includes a plurality of sections, the third region can be formed by joining ends of tubes forming each section in series. As a joining method of the above-described joint parts, there can be adopted, for example, a method of bringing the ends of resin tubes to come into contact with each other and then heat welding the contact part, or the like.

Note that the rigidity of the distal end portion of the hollow shaft 11 is preferably smaller than the rigidity of the proximal end portion of the hollow shaft 11 positioned closer to the proximal end side than the distal end portion in the longitudinal direction. The examples of such a hollow shaft 11 include, concretely, a form in which the rigidity of the hollow shaft 11 is gradually smaller from the proximal end toward the distal end, a form in which the rigidity of the hollow shaft 11 is stepwisely smaller from the proximal end toward the distal end, or the like, for example.

In the embodiment, the rigidity of the resin material is stepwisely smaller in the order of resin material A1M > resin material B1M > resin material C1M. As the combination of such resin materials AIM and C1M, the combination of polyamide and polyamide elastomer, or the combination of polyamide elastomer and polyimide is preferable.

In this manner, with the rigidity of the hollow shaft 11 made smaller toward the distal end side, it is possible to allow the catheter 1 to easily enter the inside of a curved blood vessel (improve body cavity followability).

As the size of the hollow shaft 11, it is normal that the total length is 550 mm to 1,100 mm, the proximal end of the curved part having the first, second, and third regions (proximal end side curved part W12 in the embodiment) is positioned at a part 350 mm to 900 mm from the proximal end of the hollow shaft 11, the total length of the curved part (proximal end side curved part W12) is 10 mm to 195 mm, and the total length of the third region R113 in the curved part (proximal end side curved part W12) is 5 mm to 190 mm. The outer diameter is 1.73 mm to 2.80 mm, and the inner diameter is 1.47 mm to 2.29 mm.

The connector 21 is a member for an operator to grip the catheter 1. The connector 21 is connected to the proximal end portion of the hollow shaft 11, and includes a lumen 21a connected to the lumen 11a of the hollow shaft 11, and an opening 21b formed at the proximal end of the lumen 21a. Note that the shape of the connector 21 is not especially limited as long as the effects of the present invention are not impaired, and can be any shape allowing an operator to operate the connector 12 easily, for example.

As described above, in the catheter 1 with the above-described configuration, both the composition difference between the first resin material and the third resin material and the composition difference between the second resin material and the third resin material are smaller than the composition difference between the first resin material and the second resin material, which prevents separation of connected resin tubes at the joint part. This is because the above-described configuration improves compatibility between the resin materials at each joint part and, as a result, the resins of different materials are joined firmly.

Note that the use of the catheter 1 is not especially limited as long as the effects of the present invention are not impaired, and the catheter 1 can be used as a medical catheter, for example. Particularly, the catheter 1 can be preferably applied to a guiding catheter or a microcatheter used with the hollow shaft 11 being curved. For example, in a case where the catheter 1 is used as a guiding catheter, the curved part W1 can be brought into contact with the inner wall of the body cavity such as a blood vessel, for example, for secure backup (fixing), allowing a guide wire or the like to be inserted through the lumen 11a.

### [Second Embodiment]

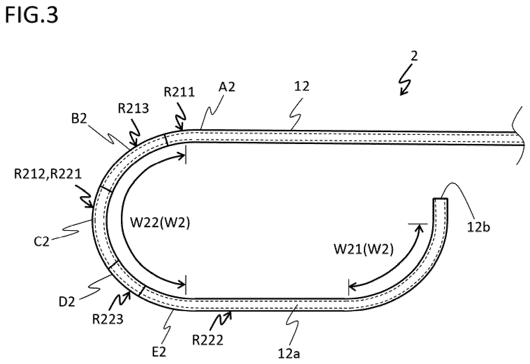

FIG. 3 is a schematic side view illustrating a second embodiment of the invention. The catheter 2 schematically includes a hollow shaft 12 and a connector 21 (not illustrated), as illustrated in FIG. 3. The catheter 2 includes the hollow shaft 12, which is different from the first embodiment. Since the configurations other than the shape of the hollow shaft 12 are the same as those of the first embodiment, the same parts are represented by the same reference signs, and detailed description thereof is omitted.

The hollow shaft 12 is a hollow-shaped shaft including at least one or more curved parts. At least one curved part of the curved parts has a first region, a second region, and a third region. In the catheter 2, the hollow shaft 12 includes two curved parts W2 of the distal end side curved part W21 and the proximal end side curved part W22, and only the proximal end side curved part W22 of these two curved parts W2 has the first to third regions at two positions.

To be more specific, one of the two first to third regions of the proximal end side curved parts W22 is a first region R211 configured by a part A2, a third region R213 configured by a part B2, and a second region R212 configured by a part C2 in this order from the proximal end side, while the other is a first region R221 configured by the part C2, a third region R223 configured by a part D2, and a second region R222 configured by a part E2. Note that in the embodiment, the second region R212 and the first region R221 are the same section (part C2).

The first region is made of the first resin material. The second region is provided closer to the distal end side in the longitudinal direction than the first region, and is made of the second resin material having a different composition from the first resin material. The third region is provided to be adjacent to the first region and the second region, and is made of the third resin material formed by mixing the first resin material and the second resin material. In the catheter 2, concretely, the first region R211 (part A2) can be made of the first resin material (resin material A2M), the third region R213 (part B2) can be made of the third resin material (resin material B2M), the second region R212 and the first region R221 (part C2) can be made of the second and first resin materials (resin material C2M), the third region R223 (D2) can be made of the third resin material (resin material D2M), and the second region R222 (E2) can be made of the second resin material (resin material E2M), for example.

Each of the resin materials A2M, C2M, E2M forming the part A2, the part C2, and the part E2 can be appropriately selected among the same resins as the resins exemplified as the first and second resin materials in the first embodiment, for example. Moreover, the resin materials B2M and D2M forming the part B2 and the part D2 may be the mixture of the resin material A2M of the part A2 and the resin material C2M of the part C2 and the mixture of the resin material C2M of the part C2 and the resin material E2M of the part E2, respectively.

Moreover, as each of the configurations of the sections in the third regions R213, R223 (part B2, part D2), it is possible to adopt the same configuration as the configuration of the section of R113 described above in the first embodiment.

Note that the rigidity of the distal end portion of the hollow shaft 12 is preferably smaller than the rigidity of the proximal end portion of the hollow shaft 12 positioned closer to the proximal end side than the distal end portion in the longitudinal direction. In the embodiment, the rigidity of the resin material is stepwisely smaller in the order of resin material A2M > resin material B2M > resin material C2M > resin material D2M > resin material E2M. Such resin materials A2M, C2M, and E2M can be appropriately selected from those exemplified as the resin materials AIM and C1M in the first embodiment, for example, on the basis of the rigidity.

In this manner, with the rigidity of the hollow shaft 12 made smaller toward the distal end side, it is possible to improve the body cavity followability, for example.

As described above, in the catheter 2 with the above-described configuration, both the composition difference between the first resin material and the third resin material and the composition difference between the second resin material and the third resin material are smaller than the composition difference between the first resin material and the second resin material, which prevents separation of connected resin tubes at the joint part.

### [Third Embodiment]

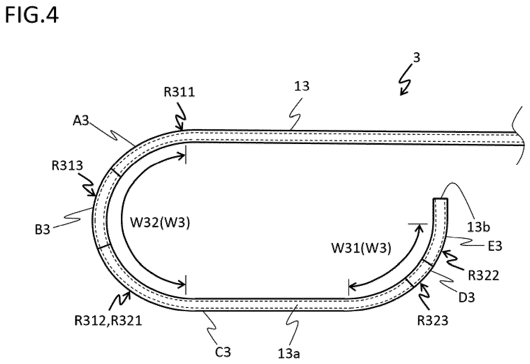

FIG. 4 is a schematic side view illustrating a third embodiment of the invention. The catheter 3 schematically includes a hollow shaft 13 and the connector 21 (not illustrated), as illustrated in FIG. 4. The catheter 3 includes a hollow shaft 13, which is different from the first embodiment. Since the configurations other than the shape of the hollow shaft 13 are the same as those of the first embodiment, the same parts are represented by the same reference signs, and detailed description thereof is omitted.

The hollow shaft 13 is a hollow-shaped shaft including one or more curved parts. At least one curved part of the curved parts has a first region, a second region, and a third region. In the catheter 3, the hollow shaft 13 includes two curved parts W3 of the distal end side curved part W31 and the proximal end side curved part W32, and each of these two curved parts W3 has the first to third regions at each position.

To be more specific, the first to third regions of the proximal end side curved parts W32 are a first region R311 configured by a part A3, a third region R313 configured by a part B3, and a second region R312 configured by a part C3, while the first to third regions of the distal end side curved parts W31 are a first region R321 configured by the part C3, a third region R323 configured by a part D3, and a second region R322 configured by a part E3. Note that in the embodiment, the second region R312 and the first region R321 are the same section (part C3).

The first region is made of the first resin material. The second region is provided closer to the distal end side in the longitudinal direction than the first region, and is made of the second resin material having a different composition from the first resin material. The third region is provided to be adjacent to the first region and the second region, and is made of the third resin material formed by mixing the first resin material and the second resin material. In the catheter 3, concretely, the first region R311 can be made of the first resin material (resin material A3M), the third region R313 can be made of the third resin material (resin material B3M), the second region R312 and the first region R321 can be made of the second and first resin material (resin material C3M), the third region R323 can be made of the third resin material (resin material D3M), and the second region R322 can be made of the second resin material (resin material E3M), for example.

Each of the resin materials A3M, C3M, E3M forming the part A1, the part C3, and the part E3 can be appropriately selected from the same resins exemplified as the first and second resin materials in the first embodiment, for example. Moreover, the resin materials B3M and D3M forming the part B3 and the part D3 may be the mixture of the resin material A3M of the part A3 and the resin material C3M of the part C3, and the mixture of the resin material C3M of the part C3 and the resin material E3M of the part E3, respectively.

Moreover, as each of the configurations of the sections in the third regions R313, R323 (part B3, part D3), it is possible to adopt the same configuration as the configuration of the section of R113 described above in the first embodiment.

Note that the rigidity of the distal end portion of the hollow shaft 13 is preferably smaller than the rigidity of the proximal end portion of the hollow shaft 13 positioned closer to the proximal end side than the distal end portion in the longitudinal direction. In the embodiment, the rigidity of the resin material is stepwisely changed in the order of resin material A3M > resin material B3M > resin material C3M > resin material D3M > resin material E3M. Such resin materials A3M, C3M, and E3M can be appropriately selected from those exemplified as the resin materials AIM, C1M in the first embodiment, for example, on the basis of the rigidity.

In this manner, with the rigidity of the hollow shaft 13 made smaller toward the distal end side, it is possible to improve the body cavity followability, for example.

As described above, in the catheter 3 with the above-described configuration, both the composition difference between the first resin material and the third resin material and the composition difference between the second resin material and the third resin material are smaller than the composition difference between the first resin material and the second resin material, which prevents separation of connected resin tubes at the joint part.

Note that the present invention is not limited to the configurations of the above-described embodiments, but is defined by the terms of the claims and is intended to include any modifications within the scope and meaning equivalent to the terms of the claims.

For example, the above-described embodiments have described the catheters 1 to 3 in which the distal ends of the curved parts having the first to third regions (proximal end side curved part W12 in the first embodiment, proximal end side curved part W22 in the second embodiment, and distal end side curved part W31 and proximal end side curved part W32 in the third embodiment) are separate from the distal ends of the hollow shafts 11 to 13. However, the catheter may include a hollow shaft (not illustrated), in which the distal end of the curved part having the first, second, and third regions is positioned at the distal end of the hollow shaft. In this manner, it is possible to improve the joint strength of the curved part at the distal end portion of the hollow shaft.

Moreover, the above-described first and second embodiments have described the catheters 1, 2 including the curved part not having the first to third regions (distal end side curved parts W11, W21). However, it is sufficient that at least one curved part of the curved parts has the first to third regions, and the catheter may also include curved parts all having the first to third regions.

Moreover, the above-described embodiments have described the catheters 1 to 3 including two curved parts. However, the catheter may include only one curved part (not illustrated), or three or more curved parts (not illustrated).

### REFERENCE SIGNS LIST

1 to 3 catheter
11 to 13 hollow shaft
R111, R211, R311, R221, R321 first region
R112, R212, R312, R222, R322 second region
R113, R213, R313, R223, R323 third region
W1 to W3 curved part

## Claims

1. A catheter, comprising:
a hollow shaft including one or more curved parts,
wherein at least one curved part of the curved parts has
a first region that is made of a first resin material,
a second region that is provided closer to a distal end side in a longitudinal direction than the first region and is made of a second resin material having a different composition from the first resin material, and
a third region that is provided to be adjacent to the first region and the second region and is made of a third resin material formed by mixing the first resin material and the second resin material.

2. The catheter according to claim 1, wherein in the third region, a mixing rate of the second resin material relative to the first resin material is gradually larger from the first region toward the second region.

3. The catheter according to claim 1 or 2, wherein a rigidity of a distal end portion of the hollow shaft is smaller than a rigidity of a proximal end portion of the hollow shaft positioned closer to a proximal end side than the distal end portion in the longitudinal direction.
